(19) Europäisches Patentamt
European Patent Office
Office européen des brevets

(11) Publication number: 0 083 977
A1

(12) EUROPEAN PATENT APPLICATION

(21) Application number: 83300075.5

(22) Date of filing: 07.01.83

(51) Int. Cl.$^3$: C 07 D 499/00
A 61 K 31/43, A 61 K 31/545
//(A61K31/545, 31/43)

(30) Priority: 11.01.82 US 338794

(43) Date of publication of application: 20.07.83 Bulletin 83/29

(84) Designated Contracting States: AT BE CH DE FR GB IT LI LU NL SE

(71) Applicant: PFIZER INC.
235 East 42nd Street
New York, N.Y. 10017(US)

(72) Inventor: Kellogg, Michael Stephen
25 Lincoln Road Waterford
New London Connecticut(US)

(74) Representative: Wood, David John et al,
Pfizer Limited Ramsgate Road
Sandwich Kent CT13 9NJ(GB)

(54) **6-Alpha-hydroxymethylpenicillanic acid sulfone as a beta-lactamase inhibitor.**

(57) 6-alpha-hydroxymethylpenicillanic acid sulfone as a useful enhancer of the effectiveness of several beta-lactam antibiotics against many beta-lactamase producing bacteria, and 6-alpha-hydroxymethylpenicillanic acid sulfone benzyl ester as a useful intermediate leading to said agent which enhance the effectiveness of beta-lactam antibiotics.

EP 0 083 977 A1

0083977

DPC 6466

# 6-alpha-Hydroxymethylpenicillanic Acid Sulfone As a beta-Lactamase Inhibitor

One of the most well-known and widely used class of antibacterial agents are the so-called beta-lactam antibiotics. These compounds are characterized in that they have a nucleus consisting of a 2-azetidinone (beta-lactam) ring fused to either a thiazolidine or a dihydro-1,3-thiazine ring. When the nucleus contains a thiazolidine ring, the compounds are usually referred to generically as penicillins, whereas when the nucleus contains a dihydrothiazine ring, the compounds are referred to as cephalosporins. Typical examples of penicillins which are commonly used in clinical practice are benzylpenicillin (pencillin G), phenoxymethylpenicillin (penicillin V), ampicillin and carbenicillin; typical examples of common cephalosporins are cephalothin, cephalexin and cefazolin.

However, despite the wide use and wide acceptance of the beta-lactam antibiotics as valuable chemotherapeutic agents, they suffer from the major drawback that certain members are not active against certain microorganisms. It is thought that in many instances this resistance of a particular microorganism to a given beta-lactam antibiotic results because the microorganism produces a beta-lactamase. The latter substances are enzymes which cleave the beta-lactam ring of penicillins and cephalosporins to give products which are devoid of antibacterial activity. However, certain substances have the ability to inhibit beta-lactamases, and when a beta-lactamase inhibitor is used in combination with a pencillin or cephalosporin it can increase or enhance the antibacterial effectiveness of the penicillin or cephalosporin against certain beta-lactamase producing microorganisms. It

is considered that there is an enhancement of antibacterial effectiveness when the antibacterial activity of a combination of beta-lactamase inhibiting substance and a beta-lactam antibiotic is significantly greater than the sum of the antibacterial activities of the individual components against beta-lactamase producing microorganisms.

The present invention relates to alpha-hydroxymethylpenicillanic acid sulfone which is a potent inhibitor of microbial beta-lactamases and which enhances the effectiveness of beta-lactam antibiotics. The invention further relates to benzyl 6-alpha-hydroxymethylpenicillanate sulfone, said ester being a useful chemical intermediate to the corresponding acid.

Pharmaceutical compositions comprising the above-mentioned 6-alpha-substituted penicillanic acid with certain beta-lactam antibiotics as well as a method for increasing the effectiveness of certain beta-lactam antibiotics in combination with the above-mentioned alpha-substituted penicillanic acid are also parts of the present invention.

Di Ninno, et al., [J. Org. Chem., 42, 2960 (1977)] have reported the synthesis of 6-beta-hydroxyalkylpenicillanic acids and the corresponding benzyl esters as potential antibacterial agents and useful intermediates, respectively.

6-Ethylpenicillanic acid and its sulfoxide derivatives are claimed as antibiotics in U.S. Patent 4,123,539.

6-alpha-Hydroxypenicillanic acid and esters thereof have been prepared from 6-diazopenicillanic acid and the corresponding esters [J. Org. Chem., 39 1444(1974)].

U.S. Patent 4,143,046 discloses 6-beta-substituted sulfonyloxypenicillanic acids as antibacterial agents, and U.S. Patent 4,287,181 claims a series of 6-beta-(1-hydroxyalkyl)penicillanic acid sulfones as potent beta-lactamase inhibitors.

The compounds of this invention are of the formula

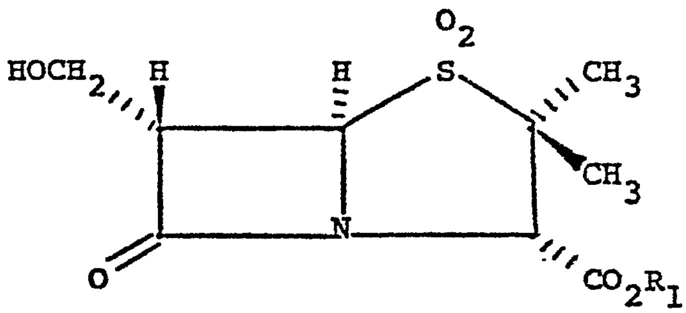

or a pharmaceutically acceptable base salt thereof, wherein $R_1$ is hydrogen or benzyl.

6-alpha-Hydroxymethypenicillanic acid sulfone ($R_1$=H) is preferred as a beta-lactamase inhibitor and benzyl 6-alpha-hydroxymethylpenicillanate sulfone ($R_1$=$C_6H_5CH_2$-) as an intermediate useful in the synthesis of said inhibitor.

The present invention also relates to a pharmaceutical composition useful for treating bacterial infections in mammals, which comprises a pharmaceutically acceptable carrier, a beta-lactam antibiotic and a compound of the formula

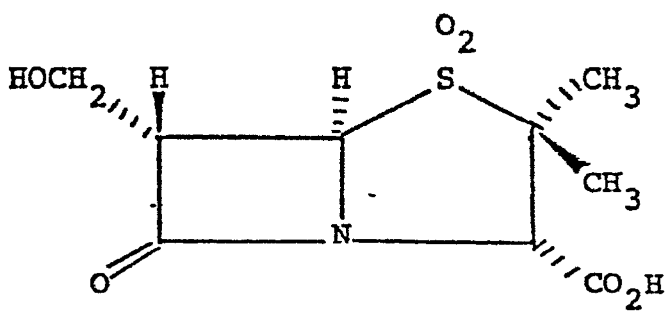

or a pharmaceutically acceptable base salt thereof.

The invention also consists of a method for increasing the effectiveness of a beta-lactam antibiotic in a mammalian subject which comprises co-administration to said subject a

beta-lactam antibiotic effectiveness increasing amount of a compound of the formula

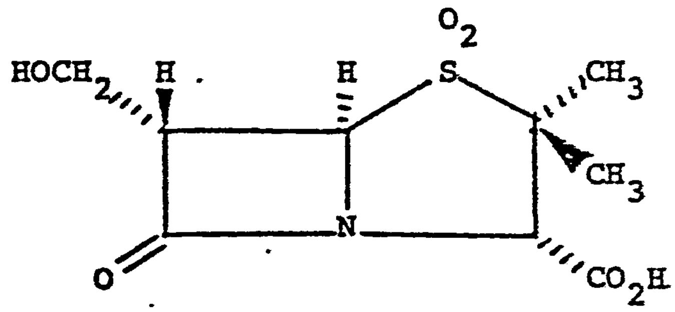

or a pharmaceutically acceptable base salt thereof.

The preferred beta-lactams whose antibiotic activity is enhanced by the 6-alpha-hydroxymethylpenicillanic acid sulfone of the present invention are selected from:
6-(2-phenylacetamido)penicillanic acid,
6-(2-phenoxyacetamido)penicillanic acid,
6-(2-phenylproprionamido)penicillanic acid,
6-(D-2-amino-2-phenylacetamido)penicillanic acid,
6-(D-2-amino-2-[4-hydroxyphenyl]acetamido)penicillanic acid,
6-(D-amino-2-[1,4-cyclohexadienyl]acetamido)penicillanic acid,
6-(1-aminocyclohexanecarboxamido)penicillanic acid,
6-(2-carboxy-2-phenylacetamido)penicillanic acid,
6-(2-carboxy-2-[3-thienyl]acetamido)penicillanic acid,
6-(D-2-[4-ethylpiperazine-2,3-dione-1-carboxamido]2-phenylacetamido)penicillanic acid,
6-(D-2-[4-hydroxy-1,5-naphthydridine-3-carboxamido]2-phenylacetamido)-penicillanic acid,
6-(D-2-sulfo-2-phenylacetamido)penicillanic acid,
6-(D-2-sulfoamino-2-phenylacetamido)penicillanic acid,
6-(D-2-[imidazolidin-2-one-1-carboxamido]2-phenylacetamido)-penicillanic acid,
6-(D-2-[3-methylsulfonylimidazolidin-2-one-1-carboxamido]-2-phenylacetamido)penicillanic acid,
6-([hexahydro-1H-azepin-1-yl]methyleneamino)penicillanic acid,

acetoxymethyl 6-(2-phenylacetamido)penicillanate,
acetoxymethyl 6-(D-2-amino-2-phenylacetamido)penicillanate,
acetoxymethyl 6-(D-2-amino-2-[4-hydroxyphenyl]acetamido)-penicillanate,
pivaloyloxymethyl 6-(2-phenylacetamido)penicillanate,
pivaloyloxymethyl 6-(D-2-amino-2-phenylacetamido)penicillanate,
pivaloyloxymethyl 6-(D-2-amino-2-[4-hydroxyphenyl]acetamido)penicillanate,
1-(ethoxycarbonyloxy)ethyl 6-(2-phenylacetamido)penicillante,
1-(ethoxycarbonyloxy)ethyl 6-(D-2-amino-2-phenylacetamido)-penicillanate,
1-(ethoxycarbonyloxy)ethyl 6-(D-2-amino-2-[4-hydroxyphenyl]-acetamido)penicillanate,
3-phthalidyl 6-(2-phenylacetamido)penicillanate,
3-phthalidyl 6-(D-2-amino-2-phenylacetamido)penicillanate,
3-phthalidyl 6-(D-2-amino-2-[4-hydroxyphenyl]acetamido)-penicillanate,
6-(2-phenoxycarbonyl-2-phenylacetamido)penicillanic acid,
6-(2-tolyloxycarbonyl-2-phenylacetamido)penicillanic acid,
6-(2-[5-indanyloxycarbonyl]-2-phenylacetamido)penicillanic acid,
6-(2-phenoxycarbonyl-2-[3-thienyl]acetamido)penicillanic acid,
6-(2-tolyloxycarbonyl-2-[3-thienyl]acetamido)penicillanic acid,
6-(2[5-indanyloxycarbonyl]-2-[3-thienyl]acetamido)penicillanic acid,
6-(2,2-dimethyl-5-oxo-4-phenyl-1-imidazolidinyl)penicillanic acid,
7-(2-[2-thienyl]acetamido)cephalosporanic acid,
7-(2-[1-tetrazolyl]acetamido-3-(2-[5-methyl-1,3,4-thiadiazolyl]thiomethyl)-3-desacetoxymethylcephalosporanic acid,

7-(D-2-formyloxy-2-phenylacetamido)-3-(5-[1-methyltetrazolyl]-thiomethyl)-3-desacetoxymethylcephalosporanic acid,

7-(D-2-amino-2-phenylacetamido)desacetoxycephalosporanic acid,

7-alpha-methoxy-7-(2-[2-thienyl]acetamido)-3-carbamoyloxy-methyl-3-desacetoxymethylcephalosporanic acid,

7-(2-cyanoacetamido)cephalosporanic acid,

7-(D-hydroxy-2-phenylacetamido)-3-(5-[1-methyltetrazolyl]-thiomethyl)-3-desacetoxymethylcephalosporanic acid,

7-(D-2-amino-2-p-hydroxyphenylacetamido)desacetoxycephalosporanic acid,

7-(2-[4-pyridylthio]acetamido)cephalosporanic acid,

7-(D-2-amino-2[1,4-cyclohexadienyl]acetamido)cephalosporanic acid,

7-(D-2-amino-]-phenylacetamido)cephalosporanic acid,

7-[D-(-)-alpha-(4-ethyl-2,3-dioxo-1-piperazinecarboxamido)-alpha-(4-hydroxyphenyl)acetamido]-3-[(1-methyl-1,2-3,4-tetrazol-5-yl)thiomethyl]-3-cephem-4-carboxylic acid,

7-(D-2-amino-2-phenylacetamido)-3-chloro-3-cephem-4-carboxylic acid,

7-[2-(2-amino-4-thiazolyl)-2-(methoximino)acetamido]cephalosporanic acid,

[6R,7R-3-carbamoyloxymethl-7(2S)-2-methoxyimino(fur-2-yl)-acetamidoceph-3-em-4-carboxylate]

7-[2-(2-aminothiazol-4-yl)acetamido]-3-[([1-2-dimethylaminoethyl)-1H-tetrazol-5-yl]thio)methyl]ceph-3-em-4-carboxylic acid,

and a pharmaceutically acceptable salt thereof.

The 6-alpha-hydroxymethylpenicillanic acid sulfone of the present application offers several advantages over the 6-beta-hydroxymethylpenicillanic acid sulfone of U.S. Patent 4,287,181.

The beta-lactamase inhibitor of the present invention is a crystalline compound as opposed to the amorphous character

of the 6-beta-hydroxyalkylpenicillanic acid sulfones of the above-mention U.S. patent.

Amorphous compounds are, in general, less desirable than is a crystalline form from the standpoint of preparation, storage and use.

A crystalline compound is considerably more stable than an amorphous form of a compound, and resists decomposing and discoloration. For pharmaceutical use, it is much easier to make up a particular dosage form using a crystalline compound as opposed to an amorphous compound. Finally, amorphous forms of a compound are frequently more hygroscopic than the crystalline form.

In addition, the 6-alpha-hydroxymethylpenicillanic acid sulfone of the present invention possesses less inherent antibacterial activity thus resulting in a greater synergistic effect when combined with a beta-lactam antibiotic.

Finally, it is well known in the art that most 6-substituted penicillanic acids possessing beta-lactamase activity are of the beta configuration. The finding of a beta-lactamase inhibitor of the potency and spectrum of activity of the compound of the present invention with the alpha configuration is unusual, if not unique.

While the present invention relates to 6-alpha-hydroxymethylpenicillanic acid sulfone and useful base salts and esters thereof, it is also meant to embrace compounds of the formula:

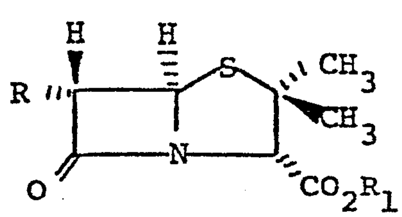

I

and

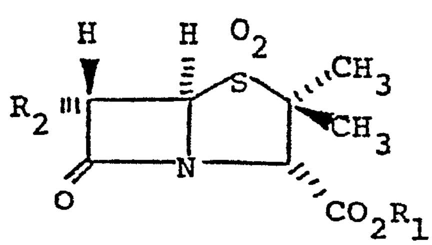

II

or a pharmaceutically acceptable base salt thereof, wherein R is alkylsulfonyloxymethyl having one to four carbon atoms

in the alkyl group, phenylsulfonyloxymethyl, substituted phenysulfonyloxymethyl wherein said substitutent is methyl, methoxy, fluoro, chloro, bromo or trifluoromethyl or 1-hydroxy-3-phenylpropyl; $R_1$ is benzyl, hydrogen or ester-forming residues readily hydrolyzable in vivo and $R_2$ is

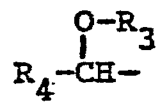

wherein $R_3$ is sulfo, hydrogen, alkoxycarbonyl of two to four carbon atoms, alkanoyl of two to eighteen carbon atoms, benzoyl, substituted benzoyl wherein said substituent is amino, methyl, methoxy, fluoro, chloro, bromo or trifluoromethyl, alkylsulfonyl of one to four carbon atoms, phenylsulfonyl or substituted phenylsulfonyl wherein said substituent is methyl, methoxy, fluoro, chloro, bromo or trifluoromethyl; and $R_4$ is hydrogen, alkyl of one to four carbon atoms, phenyl, benzyl, pyridyl or beta-phenethyl.

The beta-lactamase inhibitor of the present invention is conveniently prepared starting with benzyl 6,6-dibromopenicillanate. The condensation of formaldehyde with the enolate formed through the reaction of benzyl, 6,6-dibromopenicillanate with an organometallic reagent, such as the process taught by Di Ninno, et al., J. Org. Chem., 42, 2960 (1977), results in the formation of benzyl 6-bromo-6-hydroxymethylpenicillanate, the initial intermediate leading to the product of the present invention.

The product of this initial condensation is comprised of a diastereomeric mixture due to the asymmetric center at the 6-position of the penam nucleus.

The substituents at the 6-position are designated as alpha or beta and are so indicated in the structual formula by a broken or solid bond, respectively. The assignment of configuration is based on nuclear-magnetic-resonance spectroscopy.

Experimentally, benzyl 6,6-dibromopenicillanate in a reaction-inert solvent at -20 to -78°C. is treated with about one equivalent of t-butyl lithium or t-butyl magnesium chloride. The resulting enolate is then treated with formaldehyde and, after a short reaction period, the reaction is quenched and the product isolated by conventional means.

The initial reaction is conducted in an anhydrous reaction-inert solvent, which appreciably solubilizes the reactants without reacting to any great extent with the reactants or products under reaction conditions. It is preferred that said solvents have boiling points and freezing points compatible with reaction temperatures. Such solvents or mixtures thereof include aromatic solvents such as toluene and ethereal solvents such as tetrahydrofuran and diethyl ether.

The molar ratio of the starting penicillanate derivative and the organometallic reagent is not critical to the process. The use of a slight excess of organometallic, up to as much as ten percent above an equimolar quantity, aids in the completion of the reaction and offers no serious problems in isolating the desired product in purified form.

Moisture can effectively be kept out of the reaction by employing a nitrogen or argon atmosphere.

Reaction time is inherently dependent on concentration, reaction temperature and reactivity of the starting reagents. When the reaction is conducted at the preferred reaction temperature of -60 to -78°C. the reaction time for the formation of the enolate is about 30-45 minutes. The reaction time for the formation of the intermediate product from the aforementioned enolate and formaldehyde is about 30-60 minutes.

On completion of the reaction, the product is isolated by conventional means and the diastereomeric mixture can be separated by column chromatography. However, the nature of a subsequent reaction, which is the removal of the 6-bromo substituent, precludes the necessity for said separation of alpha and beta epimers at C-6.

The oxidation of the resulting benzyl 6-bromo-6-hydroxymethylpenicillanate to the corresponding sulfone wherein $R_1$ is benzyl is conveniently carried out using an organic peroxy acid, e.g., a peroxy-carboxylic acid such as m-chloroperbenzoic acid. The reaction is carried out by treating benzyl 6-bromo-hydroxymethylpencillanate with about 2 to about 4 equivalents and preferably about 2.2 equivalents of the oxidant in a reaction-inert solvent. Typical solvents are chlorinated hydrocarbons, such as methylene chloride, chloroform and 1,2-dichloroethane.

The oxidant and substrate are initially combined in a solvent at 0-5°C. The temperature is allowed to rise to room temperature. Reaction time is about 1-3 hours.

During isolation of the sulfone, which is a useful intermediate, the solvent is removed and the residue partitioned between water and a water immiscible solvent such as ethyl acetate. The pH of the water-organic solvent mixture is adjusted to about 7.4 and any excess peroxide can be decomposed with sodium bisulfite. The intermediate product, which is contained in the organic phase, is isolated and purified by conventional means.

Treatment of benzyl 6-bromo-hydroxymethylpenicillanate sulfone with a 5% palladium-on-calcium carbonate catalyst in a hydrogen atmosphere results in the formation of the desired 6-alpha-hydroxymethylpenicillanic acid sulfone.

The reaction is conducted at room temperature at an initial hydrogen pressure of about 40-50 psi. At these temperatures and pressures the reaction is usually complete in about 0.5-2 hours.

The reaction is carried out in a reaction-inert solvent which appreciably solubilizes the pencillanate ester without reaction to any great extent with the reactants or the product under reaction conditions. It is further preferred that said solvent be a protic solvent. The preferred solvent is 50% water-methanol.

When 5% palladium-on-calcium carbonate is employed for the reduction-debenzylation it is preferred that a two-fold weight ratio of catalyst to starting penicillanate ester reactant be employed initially or in divided portions.

On completion of the reaction, the biologically active product of the present invention wherein $R_1$ is hydrogen, is obtained by removal of the methanol, adjustment of the residual aqueous to pH 8.0 with sodium bicarbonate and extraction with a water immiscible solvent such as ethyl acetate, followed by adjustment of the aqueous to a pH of about 2 and extraction with ethyl acetate. Purification is by conventional means such as recrystalization from chloroform-ethyl acetate.

The compound of the present invention wherein $R_1$ is hydrogen, is acidic and will form salts with basic agents. Such salts are considered to be within the scope of this invention. These salts can be prepared by standard techniques, such as contacting the acidic and basic components, usually in a 1:1 molar ratio, in an aqueous, non-aqueous or partially aqueous medium, as appropriate. They are then recovered by filtration, by evaporation of the solvent, or in the case of aqueous solutions, by lyophilization, as appropriate. Basic agents which are suitably employed in salt formation belong to both the organic and inorganic types, and they include ammonia, organic amines, alkali metal hydroxides, carbonates, bicarbonates, hydrides and alkoxides, as well as alkaline earth metal hydrides, carbonates, hydrides and alkoxides. Representative examples of such bases are primary amines, such as n-propylamine, n-butylamine, cyclohexylamine, benzylamine and octylamine; secondary amines, such as diethylamine, morpholine, pyrrolidine and piperidine; tertiary amines, such as triethylamine, N-ethylpiperidine, N-methylmorpholine and 1,5-diazabicyclo[4.3.0]non-5-ene; hydroxides such as sodium hydroxide, potassium hydroxide, ammonium hydroxide and barium hydroxide; alkoxides, such as sodium ethoxide and potassium ethoxide; hydrides, such as calcium hydride and sodium hydride; carbonates, such as potassium

carbonate and sodium carbonate; bicarbonates, such as sodium bicarbonate and potassium bicarbonate; and alkali metal salts of long-chain fatty acids, such as sodium 2-ethylhexanoate.

Preferred salts of the present compound wherein $R_1$ is hydrogen are the sodium, potassium and triethylamine salts.

The compound of the present invention, wherein $R_1$ is hydrogen enhances the antibacterial effectiveness of beta-lactam antibiotics in vivo. That is, it lowers the amount of the antibiotic which is needed to protect mice against an otherwise lethal innoculum of certain beta-lactamase producing bacteria.

The ability of the compound of the present invention wherein $R_1$ is hydrogen to enhance the effectiveness of a beta-lactam antibiotic against beta-lactamase-producing bacteria makes them valuable for co-administration with beta-lactam antibiotics in the treatment of bacterial infections in mammals, particularly man. In the treatment of a bacterial infection, the said compound can be comingled with the beta-lactam antibiotic, and the two agents thereby administered simultaneously. Alternatively, the said compound can be administered as a separate agent during the course of treatment with a beta-lactam antibiotic. In some instances it will be advantageous to pre-dose the subject with the subject compound before initiating treatment with a beta-lactam antibiotic.

When using the compound of the present invention wherein $R_1$ is hydrogen to enhance the effectiveness of beta-lactam antibiotic, a mixture of said compound with the beta-lactam antibiotic is administered preferably in formulation with standard pharmaceutical carriers or diluents. A pharmaceutical composition comprising a pharmaceutically-acceptable carrier, a beta-lactam antibiotic and the compound of the present invention wherein $R_1$ is hydrogen will normally contain from about 5 to about 80 percent of the pharmaceutically acceptable carrier by weight.

When using 6-alpha-hydroxymethylpenicillanic acid sulfone in combination with another beta-lactam antibiotic, said compounds can be administered orally or parenterally, i.e., intramuscularly, subcutaneously or intraperitoneally. Although the prescribing physician will ultimately decide the dosage to be used in a human subject, the ratio of the daily dosages of the compound of the present invention ($R_1$=H) and the beta-lactam antibiotic will normally be in the range from about 1:3 to 3:1. Additionally, when using said compound in combination with another beta-lactam antibiotic, the daily oral dosage of each component will normally be in the range from about 10 to about 200 mg. per kilogram of body weight and the daily parenteral dosage of each component will normally be about 10 to about 400 mg. per kilogram of body weight. These figures are illustrative only, however, and in some case it may be necessary to use dosages outside these limits.

Typical beta-lactam antibiotics with which the compound of the present invention can be co-administered are:

6-(2-phenylacetamido)penicillanic acid,
6-(2-phenoxyacetamido)penicillanic acid,
6-(2-phenylproprionamido)penicillanic acid,
6-(D-2-amino-2-phenylacetamido)penicillanic acid,
6-(D-2-amino-2-[4-hydroxyphenyl]acetamido)penicillanic acid,
6-(D-amino-2-[1,4-cyclohexadienyl]acetamido)penicillanic acid,
6-(1-aminocyclohexanecarboxamido)penicillanic acid,
6-(2-carboxy-2-phenylacetamido)penicillanic acid,
6-(2-carboxy-2-[3-thienyl]acetamido)pencillanic acid,
6-(D-2-[4-ethylpiperazine-2,3-dione-1-carboxamido]2-phenylacetamido)penicillanic acid,
6-(D-2-[4-hydroxy-1,5-naphthydridine-3-carboxamido]2-phenylacetamido)-penicillanic acid,
6-(D-2-sulfo-2-phenylacetamido)penicillanic acid,
6-(D-2-sulfoamino-2-phenylacetamido)penicillanic acid,
6-(D-2-[imidazolidin-2-one-1-carboxamido]2-phenylacetamido)-penicillanic acid,

6-(D-2-[3-methylsulfonylimidazolidin-2-one-1-carboxamido]-2-phenylacetamido)penicillanic acid,

6-([hexahydro-1H-azepin-1-yl]methyleneamino)penicillanic acid,

acetoxymethyl 6-(2-phenylacetamido)penicillanate,

acetoxymethyl 6-(D-2-amino-2-phenylacetamido)penicillanate,

acetoxymethyl 6-(D-2-amino-2-[4-hydroxyphenyl]acetamido)-penicillanate,

pivaloyloxymethyl 6-(2-phenylacetamido)penicillanate,

pivaloyloxymethyl 6-(D-2-amino-2-phenylacetamido)penicillanate,

pivaloyloxymethyl 6-(D-2-amino-2-[4-hydroxyphenyl]acetamido)penicillanate,

1-(ethoxycarbonyloxy)ethyl 6-(2-phenylacetamido)penicillante,

1-(ethoxycarbonyloxy)ethyl 6-(D-2-amino-2-phenylacetamido)-penicillanate,

1-(ethoxycarbonyloxy)ethyl 6-(D-2-amino-2-[4-hydroxyphenyl]-acetamido)penicillanate,

3-phthalidyl 6-(2-phenylacetamido)penicillanate,

3-phthalidyl 6-(D-2-amino-2-phenylacetamido)penicillanate,

3-phthalidyl 6-(D-2-amino-2-[4-hydroxyphenyl]acetamido)-penicillanate,

6-(2-phenoxycarbonyl-2-phenylacetamido)penicillanic acid,

6-(2-tolyloxycarbonyl-2-phenylacetamido)penicillanic acid,

6-(2-[5-indanyloxycarbonyl]-2-phenylacetamido)penicillanic acid,

6-(2-phenoxycarbonyl-2-[3-thienyl]acetamido)penicillanic acid,

6-(2-tolyloxycarbonyl-2-[3-thienyl]acetamido)penicillanic acid,

6-(2[5-indanyloxycarbonyl]-2-[3-thienyl]acetamido)penicillanic acid,

6-(2,2-dimethyl-5-oxo-4-phenyl-1-imidazolidinyl)penicillanic acid,

7-(2-[2-thienyl]acetamido)cephalosporanic acid,

7-(2-[1-tetrazolyl]acetamido-3-(2-[5-methyl-1,3,4-thiadiazolyl]thiomethyl)-3-desacetoxymethylcephalosporanic acid,

7-(D-2-formyloxy-2-phenylacetamido)-3-(5-[1-methyltetrazolyl]-thiomethyl)-3-desacetoxymethylcephalosporanic acid,

7-(D-2-amino-2-phenylacetamido)desacetoxycephalosporanic acid,

7-alpha-methoxy-7-(2-[2-thienyl]acetamido)-3-carbamoyloxy-methyl-3-desacetoxymethylcephalosporanic acid,

7-(2-cyanoacetamido)cephalosporanic acid,

7-(D-hydroxy-2-phenylacetamido)-3-(5-[1-methyltetrazolyl]-thiomethyl)-3-desacetoxymethylcephalosporanic acid,

7-(D-2-amino-2-p-hydroxyphenylacetamido)desacetoxycephalosporanic acid,

7-(2-[4-pyridylthio]acetamido)cephalosporanic acid,

7-(D-2-amino-2[1,4-cyclohexadienyl]acetamido)cephalosporanic acid;

7-(D-2-amino-]-phenylacetamido)cephalosporanic acid,

7-[D-(-)-alpha-(4-ethyl-2,3-dioxo-1-piperazinecarboxamido)-alpha-(4-hydroxyphenyl)acetamido]-3-[(1-methyl-1,2-3,4-tetrazol-5-yl)thiomethyl]-3-cephem-4-carboxylic acid,

7-(D-2-amino-2-phenylacetamido)-3-chloro-3-cephem-4-carboxylic acid,

7-[2-(2-amino-4-thiazolyl)-2-(methoximino)acetamido]cephalosporanic acid,

[6R,7R-3-carbamoyloxymethl-7(2S)-2-methoxymino(fur-2-yl)-acetamidoceph-3-em-4-carboxylate]

7-[2-(2-aminothiazol-4-yl)acetamido]-3-[([1-2-dimethylaminoethyl)-1H-tetrazol-5-yl]thio)methyl]ceph-3-em-4-carboxylic acid,

and a pharmaceutically acceptable salts thereof.

As will be appreciated by one skilled in the art, some of the above beta-lactam compounds are effective when administered orally or parenterally, while others are effective only when administered by the parenteral route. When compounds of the present invention wherein $R_1$ is hydrogen

is to be used simultaneously (i.e. co-mingled) with a beta-lactam antibiotic which is effective only on parenteral administration, a combination formulation suitable for parenteral use will be required. When said compound wherein $R_1$ is hydrogen is to be used simultaneously (co-mingled) with a beta-lactam antibiotic which is effective orally or parenterally, combinations suitable for either oral or parenteral administration can be prepared. Additionally, it is possible to administer preparations of said compound where $R_1$=H orally, while at the same time administering a further beta-lactam antibiotic parenterally; and it is also possible to administer preparations of the compound of the present invention ($R_1$=H) parenterally, while at the same time administering the further beta-lactam antibiotic orally.

The following examples are provided solely for the purpose of further illustration. Nuclear magnetic resonance spectra (NMR) were measured at 60 or 100 MHz for solutions in deuterochloroform ($CDCl_3$), perdeutero dimethyl sulfoxide (DMSO-$D_6$) or deuterium oxide ($D_2O$) or are noted otherwise, and peak positions are expressed in parts per million (ppm) downfield from tetramethylsilane or sodium 2,2-dimethyl-2-silapentane-5-sulfonate. The following abbreviations for peak shapes are used: s, singlet; d, doublet; t, triplet; q, quartet; m, multiplet.

## EXAMPLE 1

### 6-alpha-Hydroxymethylpenicillanic Acid Sulfone

A. benzyl 6-bromo-6-hydroxymethylpenicillanate

A solution of 44.9 g. of benzyl 6,6-dibromopenicillanate in 600 ml. of dry tetrahydrofuran was cooled to -78°C. and 56.4 ml. of *t*-butylmagnesium chloride was added dropwise with vigorous stirring under an inert atmosphere while maintaining the temperature at -60°C. After stirring 30 min. at -78°C. the solution was treated with gaseous formaldehyde in a stream of nitrogen until five molar equivalents had been added. The reaction was quenched at -78°C.

by the addition of 5.7 ml. of acetic acid dropwise over a period of 25 min. The reaction soluton was allowed to warm to room temperature and was concentrated in vacuo. To the residue was added 200 ml. of water and 200 ml. of ethyl acetate. The organic layer was separated and the water layer extracted again with ethyl acetate. The organic phases were combined, washed successively with water (200 ml.), 5% aqueous sodium bicarbonate (200 ml.) and brine (200 ml.) and dried over magnesium sulfate. Removal of the solvent under reduced pressure provides 38.2 g. of the desired product, epimeric at C-6.

B. benzyl 6-bromo-6-hydroxymethylpenicillanate sulfone

To a solution of 500 mg. of benzyl 6-bromo-6-hydroxymethylpencillanate in 30 ml. of methylene chloride, cooled in an ice bat the 0-5°C., was added portionwise 633 mg. of 85% m-chloroperbenzoic acid over a period of 20 min. The reaction mixture was allowed to warm to room temperature and allowed to stir for about 40 min. The solvent was removed in vacuo and the residue treated with water and ethyl acetate. The pH of the mixture was adjusted to 7.4 with a saturated sodium bicarbonate solution, and the organic phase separated and treated with 30 ml. of fresh water. The pH of the mixture was adjusted to 8.2 with saturated sodium bicarbonate and the ethyl acetate layer separated and washed with a saturated sodium bicarbonate solution and a brine solution. The ethyl acetate layer was separated, dried over magnesium sulfate and evaporated to an oil, 500 mg.

C. 6-alpha-hydroxymethylpenicillanic acid sulfone

A suspension of 500 mg. of 5% palladium-on-charcoal and 500 mg. of benzyl 6-bromo-6-hydroxymethylpenicillanate sulfone in 200 ml. of 50% water-methanol was shaken in a hydrogen atmosphere at an initial pressure of 48 psi for 20 min. An additional 500 mg of fresh catalyst was added and the hydrogen pressure adjusted to 51 psi. After one hour of shaking the catalyst was filtered and the methanol removed in vacuo. The pH of the residual solution was

adjusted to 8.0 and extracted with ethyl acetate. The aqueous layer was acidified to pH2 with 6Nhydrochloric acid and the product extracted with ethyl acetate. Removal of the solvent gave 100 mg. of the desired product, which was crystallized from chloroform-ethyl acetate containing a drop of dimethyl sulfoxide, m.p. 211-212°C(dec.).

The NMR (100MHz) spectrum (DMSO-$D_6$) showed absorption at 4.93(d,1H,J=2Hz), 4.27(s,1H), 3.76(m,3H), 1.5(s,3H) and 1.4(s,3H) ppm.

CLAIMS

1. A compound of the formula:-

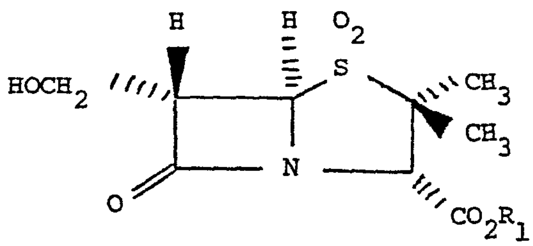

--- (I)

where $R_1$ is hydrogen or benzyl, or a pharmaceutically-acceptable base salt of said compound (I) in which $R^1$ is hydrogen.

2. The compound of claim 1, wherein $R_1$ is hydrogen.

3. The compound of claim 2 in crystalline form.

4. The compound of claim 1, wherein $R_1$ is benzyl.

5. A compound as claimed in claim 1, wherein said salt is the sodium, potassium or triethylamine salt.

6. A pharmaceutical composition useful for treating bacterial infections in mammals, which comprises a pharmaceutically acceptable carrier, a beta-lactam antibiotic, and a compound of the formula:-

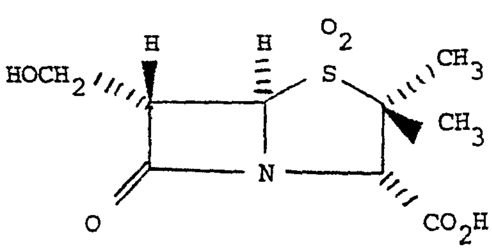

--- (IA)

or a pharmaceutically-acceptable base salt thereof.

7. A pharmaceutical composition of claim 6 wherein said beta-lactam antibiotic is selected from:

6-(2-phenylacetamido)penicillanic acid,

6-(2-phenoxyacetamido)penicillanic acid,

6-(2-phenylproprionamido)penicillanic acid,

6-(D-2-amino-2-phenylacetamido)penicillanic acid,

6-(D-2-amino-2-[4-hydroxyphenyl]acetamido)penicillanic acid,

6-(D-amino-2-[1,4-cyclohexadienyl]acetamido)penicillanic acid,

6-(1-aminocyclohexanecarboxamido)penicillanic acid,

6-(2-carboxy-2-phenylacetamido)penicillanic acid,

6-(2-carboxy-2-[3-thienyl]acetamido)penicillanic acid,

6-(D-2-[4-ethylpiperazine-2,3-dione-1-carboxamido]2-phenylacetamido)penicillanic acid,

6-(D-2-[4-hydroxy-1,5-naphthydridine-3-carboxamido]2-phenylacetamido)-penicillanic acid,

6-(D-2-sulfo-2-phenylacetamido)penicillanic acid,

6-(D-2-sulfoamino-2-phenylacetamido)penicillanic acid,

6-(D-2-[imidazolidin-2-one-1-carboxamido]-2-phenylacetamido)-penicillanic acid,

6-(D-2-[3-methylsulfonylimidazolidin-2-one-1-carboxamido]-2-phenylacetamido)penicillanic acid,

6-([hexahydro-1H-azepin-1-yl]methyleneamino)penicillanic acid,

acetoxymethyl 6-(2-phenylacetamido)penicillanate,

acetoxymethyl 6-(D-2-amino-2-phenylacetamido)penicillanate,

acetoxymethyl 6-(D-2-amino-2-[4-hydroxyphenyl]acetamido)-penicillanate,

pivaloyloxymethyl 6-(2-phenylacetamido)penicillanate,

pivaloyloxymethyl 6-(D-2-amino-2-phenylacetamido)penicillanate,

pivaloyloxymethyl 6-(D-2-amino-2-[4-hydroxyphenyl]acetamido) penicillanate,

1-(ethoxycarbonyloxy)ethyl 6-(2-phenylacetamido)penicillanate,

1-(ethoxycarbonyloxy)ethyl 6-(D-2-amino-2-phenylacetamido)-penicillanate,

1-(ethoxycarbonyloxy)ethyl 6-(D-2-amino-2-[4-hydroxyphenyl]-acetamido)penicillanate,

3-phthalidyl 6-(2-phenylacetamido)penicillanate,

3-phthalidyl 6-(D-2-amino-2-phenylacetamido)penicillanate,

3-phthalidyl 6-(D-2-amino-2-[4-hydroxyphenyl]acetamido)-penicillanate,

6-(2-phenoxycarbonyl-2-phenylacetamido)penicillanic acid,

6-(2-tolyloxycarbonyl-2-phenylacetamido)penicillanic acid,

6-(2-[5-indanyloxycarbonyl]-2-phenylacetamido)penicillanic acid,

6-(2-phenoxycarbonyl-2-[3-thienyl]acetamido)penicillanic acid,

6-(2-tolyloxycarbonyl-2-[3-thienyl]acetamido)penicillanic acid,

6-(2-[5-indanyloxycarbonyl]-2-[3-thienyl]acetamido)penicillanic acid,

6-(2,2-dimethyl-5-oxo-4-phenyl-1-imidazolidinyl)penicillanic acid,

7-(2-[2-thienyl]acetamido)cephalosporanic acid,

7-(2-[1-tetrazolyl]acetamido-3-(2-[5-methyl-1,3,4-thiadiazolyl] thiomethyl)-3-desacetoxymethylcephalosporanic acid,

7-(D-2-formyloxy-2-phenylacetamido)-3-(5-[1-methyltetrazolyl]-thiomethyl)-3-desacetoxymethylcephalosporanic acid,

7-(D-2-amino-2-phenylacetamido)desacetoxycephalosporanic acid,

7-alpha-methoxy-7-(2-[2-thienyl]acetamido)-3-carbamoyloxy-methyl-3-desacetoxymethylcephalosporanic acid,

7-(2-cyanoacetamido)cephalosporanic acid,

7-(D-hydroxy-2-phenylacetamido)-3-(5-[1-methyltetrazolyl]-thiomethyl)-3-desacetoxymethylcephalosporanic acid,

7-(D-2-amino-2-p-hydroxyphenylacetamido)desacetoxycephalosporanic acid,

7-(2-[4-pyridylthio]acetamido)cephalosporanic acid,

7-(D-2-amino-2-[1,4-cyclohexadienyl]acetamido)cephalosporanic acid,

7-(D-2-amino-2-phenylacetamido)cephalosporanic acid,

7-[D-(-)-alpha-(4-ethyl-2,3-dioxo-1-piperazinecarboxamido)-alpha-(4-hydroxyphenyl)acetamido]-3-[(1-methyl-1,2,3,4-tetrazol-5-yl)thiomethyl]-3-cephem-4-carboxylic acid,

7-(D-2-amino-2-phenylacetamiod)-3-chloro-3-cephem-4-carboxylic acid,

7-[2-(2-amino-4-thiazolyl)-2-(methoximino)acetamido] cephalosporanic acid,

[6R,7R-3-carbamoyloxymethyl-7(2S)-2-methoxyimino(fur-2-yl)-acetamidoceph-3-em-4-carboxylatel,

7-[2-(2-aminothiazol-4-yl)acetamido]-3-[([1-2-dimethylaminoethyl)-1H-tetrazol-5-ylthio)methyl]ceph-3-em-4-carboxylic acid,

and their pharmaceutically-acceptable salts.

0083977

CLAIMS FOR AT

1. A process for preparing a compound of the formula

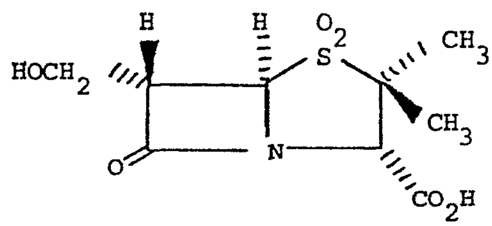

characterized by contacting a compound of the formula

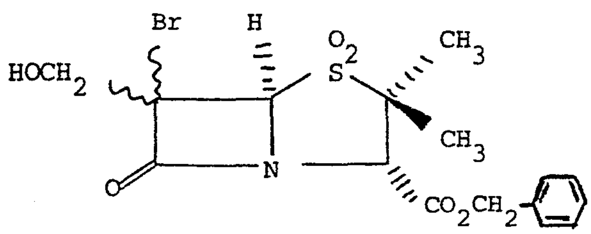

with hydrogen and a catalyst and, if desired, making a pharmaceutically acceptable base salt thereof.

2. The process of claim 1 wherein the catalyst is palladium-on-calcium carbonate and the reaction temperature is room temperature.

3. A process for preparing a compound of the formula

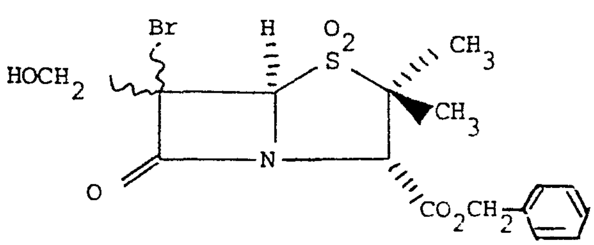

characterised by reacting a compound of the formula

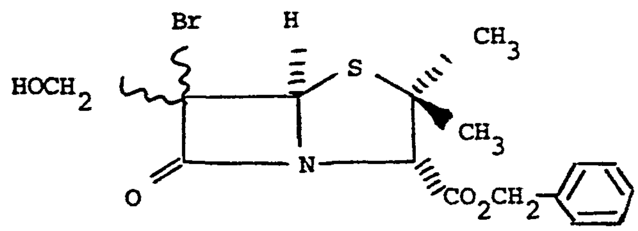

with an oxidizing agent.

4. The process of claim 3 wherein said oxidizing agent is m-chloroperbenzoic acid.

European Patent Office

**0083977**

Application number

# EUROPEAN SEARCH REPORT

EP 83 30 0075

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 041 047 (CIBA-GEIGY) *Page 13, paragraph 3; page 52, example 6; claims 1-19* | 1-7 | C 07 D 499/00<br>A 61 K 31/43<br>A 61 K 31/545//<br>(A 61 K 31/545<br>A 61 K 31/43 ) |
| A | GB-A-2 053 220 (BAYER) *Claims 1,5,18,26-29* | 1,6 | |
| | | | TECHNICAL FIELDS SEARCHED (Int. Cl. 3) |
| | | | C 07 D 499/00<br>A 61 K 31/00 |

The present search report has been drawn up for all claims

| Place of search | Date of completion of the search | Examiner |
|---|---|---|
| THE HAGUE | 07-04-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document